# EUROPEAN PATENT APPLICATION

(11) **EP 3 372 615 A1**
(43) Date of publication of application: **12.09.2018**
(21) Application number: 17159470.8
(22) Date of filing: 06.03.2017
(51) Int. Cl.: C07K 16/28

(54) **COMPOSITION COMPRISING AVELUMAB**

(71) Applicant: Merck Patent GmbH, 64293 Darmstadt (DE)
(72) Inventor: Terlizzese, Mariagrazia, 00125 Roma RM (IT); Cavaliere, Francesca, 00177 Roma RM (IT); Pezzotti, Anna R., 00132 Roma RM (IT); De Rossi, Elio, 00060 Capena RM (IT)
(74) Representative: Merck Serono S.A. Intellectual Property

(57) **Abstract**

The present invention relates to avelumab antibody compositions with an elevated deamidation level, as well as methods for using such compositions to treat a disorder, e.g., a disorder in which the interaction between PD-1 and PD-L1 is detrimental.

SEQUENCE LISTING

<110>  Merck Patent GmbH

<120>  COMPOSITION COMPRISING AVELUMAB

<130>  P 17/042

<160>  4

<170>  PatentIn version 3.5


<210>  1
<211>  216
<212>  PRT
<213>  Artificial Sequence

<220>
<223>  from human Fab library

<220>
<221>  CHAIN
<222>  (1)..(216)
<223>  A09-246-2 complete light chain

<220>
<221>  CHAIN
<222>  (1)..(22)
<223>  A09-246-2 light chain framework region 1

<220>
<221>  CHAIN
<222>  (1)..(110)
<223>  A09-246-2 light chain variable region

<220>
<221>  BINDING
<222>  (23)..(36)
<223>  A09-246-2 light chain complementarity determining region 1

<220>
<221>  CHAIN
<222>  (37)..(51)
<223>  A09-246-2 light chain framework region 2

<220>
<221>  BINDING
<222>  (52)..(58)
<223>  A09-246-2 light chain complementarity determining region 2

<220>
<221>  CHAIN
<222>  (59)..(90)
<223>  A09-246-2 light chain framework region 3

<220>
<221>  BINDING
<222>  (91)..(100)
<223>  A09-246-2 light chain complementarity determining region 3

<220>
<221>  CHAIN

<222>  (101)..(110)
<223>  A09-246-2 light chain framework region 4

<220>
<221>  CHAIN
<222>  (111)..(216)
<223>  A09-246-2 light chain constant domain

<400>  1

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Arg Val Phe Gly Thr Gly Thr Lys Val Thr Val Leu Gly Gln
            100                 105                 110

Pro Lys Ala Asn Pro Thr Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Gly Ser Pro Val Lys
145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser

210                 215

<210>  2
<211>  450
<212>  PRT
<213>  Artificial Sequence

<220>
<223>  from human Fab library

<220>
<221>  CHAIN
<222>  (1)..(450)
<223>  A09-246-2 complete heavy chain

<220>
<221>  CHAIN
<222>  (1)..(30)
<223>  A09-246-2 heavy chain framework region 1

<220>
<221>  CHAIN
<222>  (1)..(120)
<223>  A09-246-2 heavy chain variable region

<220>
<221>  BINDING
<222>  (31)..(35)
<223>  A09-246-2 heavy chain complementarity determining region 1

<220>
<221>  CHAIN
<222>  (36)..(49)
<223>  A09-246-2 heavy chain framework region 2

<220>
<221>  BINDING
<222>  (50)..(66)
<223>  A09-246-2 heavy chain complementarity determining region 2

<220>
<221>  CHAIN
<222>  (67)..(98)
<223>  A09-246-2 heavy chain framework region 3

<220>
<221>  BINDING
<222>  (99)..(109)
<223>  A09-246-2 heavy chain complementarity determining region 3

<220>
<221>  CHAIN
<222>  (110)..(120)
<223>  A09-246-2 heavy chain framework region 4

<220>
<221>  CHAIN
<222>  (121)..(218)
<223>  A09-246-2 heavy chain constant domain 1

<220>

<221>  CHAIN
<222>  (219)..(233)
<223>  A09-246-2 hinge region

<220>
<221>  CHAIN
<222>  (234)..(450)
<223>  A09-246-2 heavy chain constant domain 2 and 3

<400>  2

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ile Met Met Trp Val Arg Gln Ala Pro Gly Lys Gly Leu Glu Trp Val
        35                  40                  45

Ser Ser Ile Tyr Pro Ser Gly Gly Ile Thr Phe Tyr Ala Asp Thr Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ile Lys Leu Gly Thr Val Thr Thr Val Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  3
<211>  14
<212>  PRT
<213>  Artificial Sequence

<220>
<223>  from human Fab library

<400>  3

Thr Gly Thr Ser Ser Asp Val Gly Gly Tyr Asn Tyr Val Ser
1               5                   10

<210>  4
<211>  7
<212>  PRT
<213>  Artificial Sequence

<220>
<223>  from human Fab library

<400>  4

Asp Val Ser Asn Arg Pro Ser
1               5

## Description

### INTRODUCTION

The present invention relates to avelumab antibody compositions with an elevated deamidation level, as well as methods for using such compositions to treat a disorder, e.g., a disorder in which the interaction between PD-1 and PD-L1 is detrimental.

### BACKGROUND

Large scale production of a therapeutic protein yields a heterogeneous population of target protein. The produced protein can be subject to various modifications, which, dependent on the nature of the particular modification, can affect the biological function and stability of it.

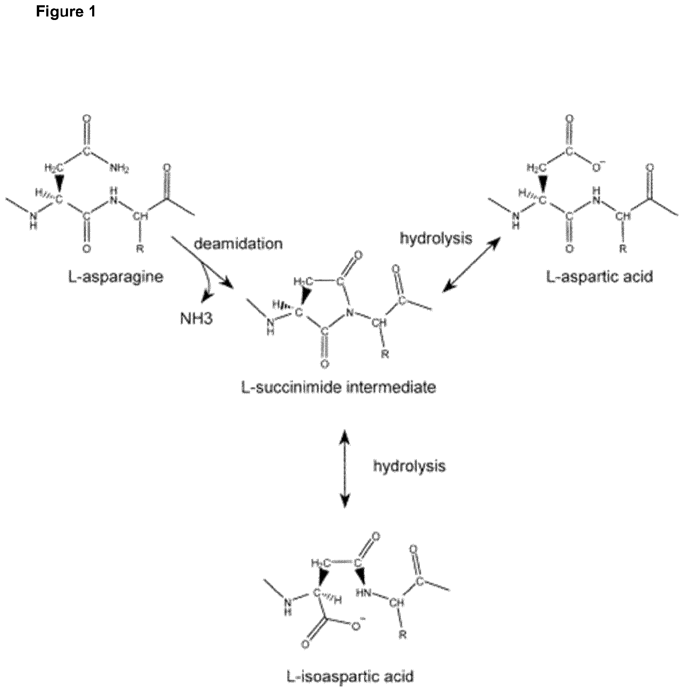

Deamidation of asparagine (Asn or N) residues is one of the most common posttranslational modifications occurring in therapeutic proteins produced using recombinant DNA technology. In this reaction, the Asn residue is, via a succinimide intermediate (Asu), converted into isoaspartate (isoAsp or isoD) or aspartate (Asp or D) at an approximate 3:1 ratio (Figure 1).

Deamidation of an Asn residue is rate-dependent on various factors, including the flanking amino acids, solvent dielectric constant and solvent viscosity, temperature, solution pH and the higher-order structure of the protein (Wakankar and Borchardt, J Pharm Sci. 2006 Nov;95(11):2321-36).

There have been various reports of Asn deamidation negatively impacting the binding affinity of antibodies.

Harris et al. showed that the deamidation of Asn30 in the light chain (LC) CDR1 of the mAb Herceptin reduced the binding potency of this antibody to 70% (J Chromatogr B Biomed Sci Appl. 2001 Mar 10;752(2):233-45).

Deamidation of an Asn residue in the CDR1 loop of the LC (Asn33) of an IgG1 mAb was shown to reduce antigen binding by 40-60% (Vlasak et al., Anal Biochem. 2009 Sep 15;392(2):145-54).

In another example of a humanized recombinant IgG1 mAb, Asn55 of the heavy chain (HC) CDR2 was found to be prone to deamidation to isoAsp and Asp, leading to a 14-fold reduction in binding activity (Huang et al., Anal Chem. 2005 Mar 1;77(5):1432-9).

A case of succinimide formation at Asn55 of the HC CDR2 of an IgG1 mAb resulting in 70% drop in potency has also been reported (Yan et al., J Pharm Sci. 2009 Oct;98(10):3509-21).

Yuang et al. reported that the deamidation of Asn33 of the LC of an IgG2 antibody correlates with a loss of potency, leading to an almost complete loss of potency after incubation of the antibody for one month at 40°C (MAbs. 2013 Sep-Oct;5(5):787-94).

In view of the potentially deleterious effects of deamidation on the binding affinity of therapeutic proteins, there has been a desire to minimize this post-translational modification, and ways of protecting proteins against deamidation both during and after manufacturing have been developed (Gervais, J Chem Technol Biotechnol 2016;91:569-575).

For instance, Kaneko et al. describe that the day of harvest affects the amount of deamidated species of a mAb (J Biosci Bioeng. 2010 Mar;109(3):281-7). In this study, the different variants of the mAb were resolved by ion-exchange (IEX) chromatography after different days of harvest. The peak corresponding to the deamidated form of the mAb increased from 1.06% at day 4 of harvest to 7.71% at day 10 of harvest.

This data is in line with data presented by Dengl et al., which further suggests that, e.g., the pH and temperature affect the degree of deamidation of the investigated mAb (Pharm Res. 2013 May;30(5):1380-99).

Also the supplementation of various additives into mammalian cell culture media has been reported to significantly reduce the accumulation of deamidated proteins (Hossler et al., Biotechnol Prog. 2015 Jul-Aug;31(4):1039-52; Brühlmann et al., Biotechnol Prog. 2015 May-Jun;31(3):615-29).

After manufacture, deamidation can be minimized by the storage conditions selected, including the choice of an appropriate formulation. Cleland et al. describe that deamidation of lyophilized formulations of the humanized mAb against human EGFR-2 (rhuMAb HER2) can be inhibited during storage for 3 months at 40°C by choosing a sugar-to-protein molar ratio of 360:1 (J Pharm Sci. 2001 Mar;90(3):310-21).

One therapeutic protein that is currently in clinical development is the anti-PD-L1 antibody avelumab (WO2013079174A1).

The programmed death 1 (PD-1) receptor and PD-1 ligands 1 and 2 (PD-L1, PD-L2) play integral roles in immune regulation. Expressed on activated T cells, PD-1 is activated by PD-L1 and PD-L2 expressed by stromal cells, tumor cells, or both, initiating T-cell death and localized immune suppression, potentially providing an immune-tolerant environment for tumor development and growth (Dong et al., Nat Med. 1999 Dec;5(12):1365-9; Freeman et al., J Exp Med. 2000 Oct 2;192(7):1027-34; Dong et al., Nat Med. 2002 Aug;8(8):793-800; Topalian et al., Curr Opin Immunol. 2012 Apr;24(2):207-12). Conversely, inhibition of this interaction can enhance local T-cell responses and mediate antitumor activity in nonclinical animal models (Dong et al., Nat Med. 2002 Aug;8(8):793-800; Iwai et al., Proc Natl Acad Sci USA. 2002 Sep 17;99(19):12293-7). In the clinical setting, treatment with antibodies that block the PD-1 - PD-L1 interaction have been reported to produce objective response rates of 7% to 38% in patients with advanced or metastatic solid tumors, with tolerable safety profiles (Hamid et al., N Engl J Med. 2013 Jul 11;369(2):134-44; Brahmer et al., N Engl J Med. 2012 Jun 28;366(26):2455-65; Topalian et al., N Engl J Med. 2012 Jun 28;366(26):2443-54; Herbst et al., Nature. 2014 Nov 27;515(7528):563-7). Notably, responses appeared prolonged, with durations of 1 year or more for the majority of patients.

Avelumab (also known as MSB0010718C) is a fully human monoclonal antibody of the immunoglobulin (Ig) G1 isotype. Avelumab selectively binds to PD-L1 and competitively blocks its interaction with PD-1.

Compared with anti-PD-1 antibodies that target T-cells, avelumab targets tumor cells, and therefore is expected to have fewer side effects, including a lower risk of autoimmune-related safety issues, since blocking of PD-L1 leaves the PD-L2 - PD-1 pathway intact to promote peripheral self-tolerance (Latchman et al., Nat Immunol. 2001 Mar;2(3):261-8).

Avelumab is currently being tested in the clinic in a number of cancer types including non-small cell lung cancer, urothelial carcinoma, mesothelioma, Merkel cell carcinoma, gastric or gastroesophageal junction cancer, ovarian cancer, and breast cancer.

Further medical uses of Avelumab are described in WO2016137985, WO2016181348, WO2016205277, PCT/US2016/053939, U.S. patent application Ser. No.'s 62/423,358.

WO2013079174 also describes in section 2.4 a human aqueous formulation of an antibody having the amino acid sequence of Avelumab. This formulation comprises the antibody in a concentration of 10 mg/ml, methionine as an antioxidant and has a pH of 5.5. Avelumab formulations not comprising an antioxidant are described in PCT/EP2016/002040.

### SUMMARY OF THE INVENTION

The present inventors surprisingly found that there is no negative correlation between the deamidation level of an avelumab composition and its cell binding activity.

Accordingly, in one aspect, the invention provides a composition comprising avelumab, wherein a fraction of the avelumab molecules is deamidated.

In a further aspect, the invention provides a composition comprising avelumab, wherein the proportion of deamidated avelumab variants is more than 2%, more than 4%, more than 6%, more than 8%, more than 10% or more than 12%.

In a further aspect, the invention provides a composition comprising avelumab, wherein the proportion of deamidated avelumab variants is within a range of 2-50%, 4-40%, 6-30%, 8-30%, 10-25%, 12-20%, or 12-16%.

The composition of the invention comprising deamidated avelumab variants preferably has a low molecular weight (LMW) species content of less than 15%, less than 10%, less than 8%, less than 6% or less than 4%. In some embodiments, the LMW species content is in the range of 0.1-10%, 2-6% or 3-5%.

Furthermore, the composition of the invention comprising deamidated avelumab variants preferably has less than 15%, less than 10%, less than 8% or less than 6% avelumab variants that are oxidated at HC Met255. In some embodiments, the content of avelumab variants that are oxidated at HC Met255 is in the range of 0.1-10%, 1-8% or 3-6%.

In some embodiments, the avelumab composition has 8-25% deamidated variants, 0.1-10% variants oxidated at Met255 of the heavy chain and 0.1-10% LMW species. In other embodiments, the avelumab composition has 12-20% deamidated variants, 1-8% variants oxidated at Met255 of the heavy chain and 2-6% LMW species. In yet other embodiments, the avelumab composition has 12-16% deamidated variants, 3-6% variants oxidated at Met255 of the heavy chain and 3-5% LMW species.

In some embodiments, the avelumab composition has 8-25% deamidated variants, less than 10% variants oxidated at Met255 of the heavy chain and less than 10% LMW species. In other embodiments, the avelumab composition has 8-25% deamidated variants, less than 6% variants oxidated at Met255 of the heavy chain and less than 6% LMW species. In other embodiments, the avelumab composition has 12-20% deamidated variants, less than 8% variants oxidated at Met255 of the heavy chain and less than 6% LMW species. In yet other embodiments, the avelumab composition has 12-16% deamidated variants, less than 6% variants oxidated at Met255 of the heavy chain and less than 5% LMW species.

In some embodiments, the avelumab composition has more than 6% deamidated variants, less than 10% variants oxidated at Met255 of the heavy chain and less than 10% LMW species. In other embodiments, the avelumab composition has more than 8% deamidated variants, less than 8% variants oxidated at Met255 of the heavy chain and less than 6% LMW species. In yet other embodiments, the avelumab composition has more than 10% deamidated variants, less than 6% variants oxidated at Met255 of the heavy chain and less than 5% LMW species.

In some embodiments, the avelumab composition of the invention has less than 20%, less than 15%, less than 10%, or less than 7.5% of the avelumab molecules afucosylated.

In some embodiments, less than 20%, less than 10%, less than 5%, or less than 3% of the avelumab molecules of the avelumab composition of the invention have high mannose N-glycans with five or more mannose residues.

Any features, including optional, suitable, and preferred features, described in relation to any particular aspect of the invention may also be features, including optional, suitable and preferred features, in relation to other aspects of the present invention.

### BRIEF DESCRIPTION OF THE DRAWINGS

For a better understanding of the invention, and to show how embodiments of the same are put into effect, reference is now made, by way of example, to the following diagrammatic drawings:
Figure 1 depicts the degradative pathway for Asn deamidation (from Chelius et al., Anal Chem. 2005 Sep 15;77(18):6004-11).

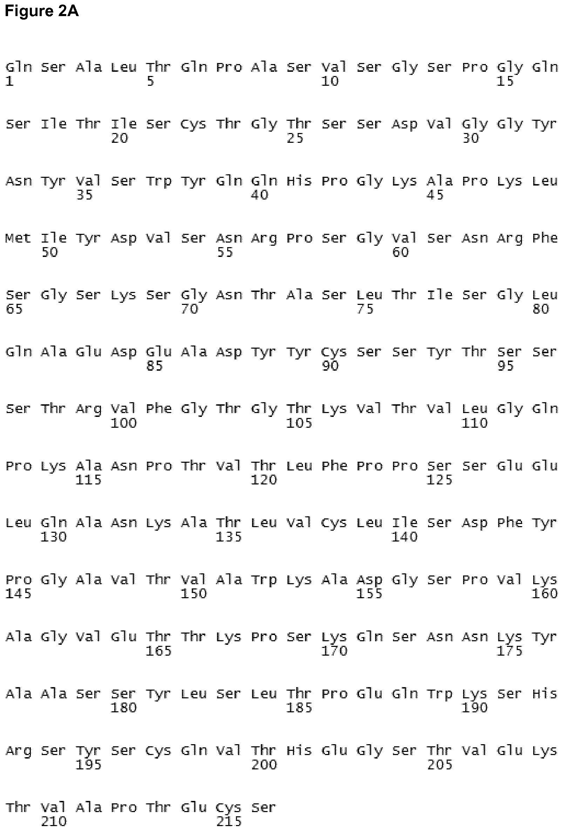

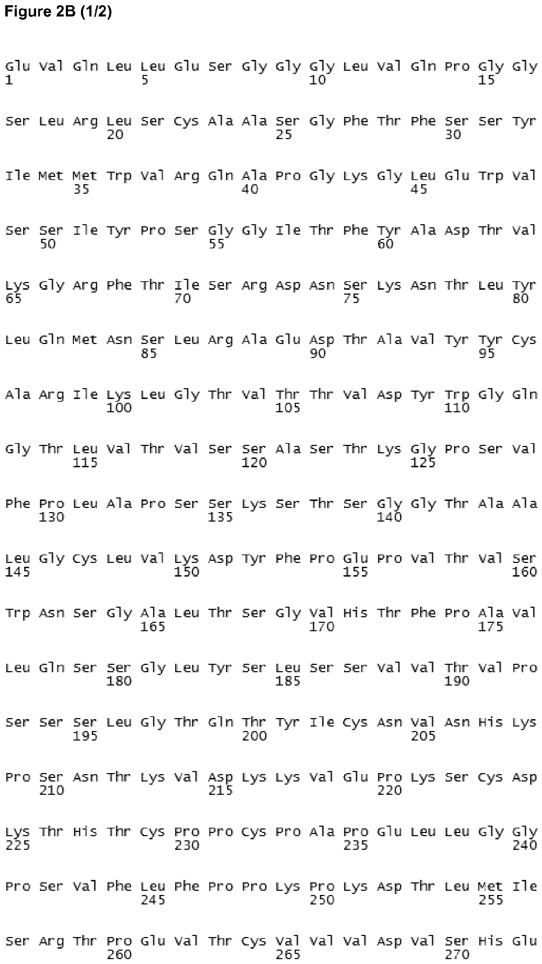

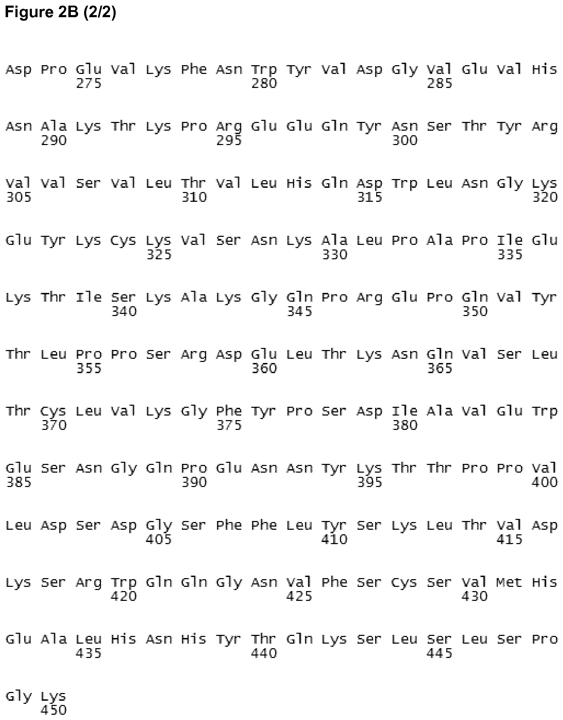

Figure 2 depicts the light chain (Figure 2A) and heavy chain protein sequence of avelumab (Figure 2B).
Figure 3 shows a typical IEX profile of a blank sample treated with carboxypeptidase B.
Figure 4 shows a typical IEX profile of an avelumab sample treated with carboxypeptidase B in an overlay with a blank sample treated with carboxypeptidase B.
Figure 5 shows a typical IEX profile of an avelumab sample artificially deamidated by incubating it for 4 weeks at 50°C and treated with carboxypeptidase B in an overlay with a blank sample treated with carboxypeptidase B.
Figures 6 and 7 show correlations between the level of deamidation and the cell binding activity of avelumab.

### DETAILED DESCRIPTION OF THE INVENTION

Avelumab has two potential deamidation sites in its CDR regions. Asn33 is found inside the light chain CDR1 sequence TGTSSDVGGYNYVS (SEQ ID NO: 3) and Asn55 is found inside the light chain CDR2 sequence DVSNRPS (SEQ ID NO: 4). The present inventors assessed the impact of deamidation on avelumab binding to the cell surface of PD-L1 expressing cells. Surprisingly, the inventors found that the overall Asn deamidation level of avelumab has no negative correlation with its cell binding activity, whereas many prior art reports revealed a negative impact of deamidation in the CDR regions on binding potency.

In line with these results, it has been found during clinical studies that avelumab compositions having 10-25% deamidated variants show a high therapeutic effectiveness.

### Definitions

Unless otherwise stated, the following terms used in the specification and claims have the following meanings set out below.

As used herein, the term "avelumab" refers to a fully human anti-PD-L1 IgG1 monoclonal antibody (also known as MSB0010718C) and described in WO2013079174A1 as antibody A09-246-2. Avelumab, as used herein, is not limited in relation to its post-translational modifications and includes biosimilar versions of the molecule.

Most preferably, avelumab comprises the light chain sequence of SEQ ID NO: 1 and the heavy chain sequence of SEQ ID NO: 2, preferably including post-translational variants thereof. Post-translational variants include, for instance, deamidated variants and variants having the C-terminal lysine of the heavy chain cleaved off.

The term "biosimilar" (also know as follow-on biologics) is well known in the art, and the skilled person would readily appreciate when a drug substance would be considered a biosimilar of avelumab. A biosimilar is a biological medicine that is similar to another biological medicine that has already been authorised for use (also known as the reference medicine). For biosimilars to be approved, it must be shown that the medicine is similar to the reference medicine and does not have any meaningful differences from the reference medicine in terms of quality, safety or efficacy. Since biologics have a high degree of molecular complexity, and are generally sensitive to changes in manufacturing processes (e.g. if different cell lines are used in their production), and since subsequent follow-on manufacturers generally do not have access to the originator's molecular clone, cell bank, know-how regarding the fermentation and purification process, nor to the active drug substance itself (only the innovator's commercialized drug product), any "biosimilar" is unlikely to be exactly the same as the innovator drug product.

Herein, the term "deamidated variant" means a variant of avelumab, which has a deamidation in at least one position. In a deamidation reaction, the amide group from the the side-chain residue of Asn or Gin is lost. The end product of the deamidation reaction has a lowered isoelectric point (pi), since the charge of the deamidated residue changes from neutral to negative. Deamidation of Asn residues is much more common than deamidation of Gin residues. The deamidation of a residue in avelumab causes this variant to differ in sequence in this particular residue, as compared to the unmodified form of avelumab. The proportion of the deamidated variants relates to the proportion of variants in relation to the total intact molecule, i.e. avelumab as described above, which does not include, e.g., LMW species.

Herein, the term "oxidated variant" refers to a variant of avelumab, which is oxidated at methionine 255 of the heavy chain. Each avelumab molecule has two heavy chains and may be oxidated at none, one or two of the heavy chains. The proportion of oxidated variants refers to the proportion of HC Met255 residues that are oxidated in relation to all HC Met255 residues.

Herein, the term "low molecular weight species" refers to all those avelumab species detected at a molecular weight lower than intact molecule.

Herein, the term "pharmaceutically acceptable carrier" refers to one or more materials that do not interfere with the effectiveness of the biological activity of the active ingredient and that are not toxic to the patient when administered in typical amounts. Such carriers are added to falcilitate the application of the active ingredient to the patient.

It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, *i.e.,* arresting, reducing or delaying the development of the disease or a relapse thereof (in case of maintenance treatment) or at least one clinical or subclinical symptom thereof, or (3) relieving or attenuating the disease, *i.e.,* causing regression of the state, disorder or condition or at least one of its clinical or subclinical symptoms.

### Compositions having elevated deamidation levels

The present invention provides an avelumab composition wherein a fraction of the avelumab molecules is deamidated. In other words, a subset of the avelumab molecules of the avelumab composition of the invention is deamidated in one or more positions, preferably at least in LC Asn33 and/or LC Asn55. It has been found that an avelumab composition with an increased amount of deamidated variants correlates with an increase in PD-L1 binding on the cell surface.

In some embodiments, the avelumab composition has more than 2%, more than 4%, more than 6%, more than 8%, more than 10% or more than 12% of deamidated variants.

Avelumab batches with deamidated variants in the range of about 10-25% were not only found to show high cell binding activity (confirmed by high binding affinity to PD-L in a Biacore assay), but also high ADCC activity, which is a suggested further mode of action of avelumab. Multiple batches within this deamidation range were tested in clinical trials and the treatment therewith was found to be safe and efficacious. In line with this, the range of deamidated variants in the avelumab composition of the invention can be 2-50%, 4-40%, 6-30%, 8-30%, 10-25% or 12-20%.

The LMW species content of the avelumab batches that were tested in clinical trials was less than 10%. Accordingly, the composition of the invention preferably has a LMW species content of less than 15%, less than 10%, less than 8%, less than 6% or less than 5%. In some embodiments, the LMW species content is in the range of 0.1-10%, 2-6% or 3-5%. The content of avelumab variants with an oxidized heavy chain methionine (Met) 255 in the tested batches was less than 10%. Accordingly, the composition of the invention preferably has less than 15%, less than 10%, less than 8% or less than 6% avelumab variants that are oxidated at HC Met255. In some embodiments, the content of avelumab variants that are oxidated at HC Met255 is in the range of 0.1-10%, 1-8% or 3-6%.

In some embodiments, the avelumab composition has 8-25% deamidated variants, less than 10% variants oxidated at Met255 of the heavy chain and less than 10% LMW species. Such composition was found to have a high ADCC level, as well as high cell binding activity.

In some embodiments, the avelumab composition of the invention has less than 20%, less than 15%, less than 10%, or less than 7.5% of the avelumab molecules afucosylated.

In some embodiments, less than 20%, less than 10%, less than 5%, or less than 3% of the avelumab molecules of the avelumab composition of the invention have high mannose N-glycans with five or more mannose residues.

The composition of the invention is preferably stored after its manufacture below 12°C, below 10°C, or below 8°C until it is to be administered to the patient. In some embodiments, the composition of the invention is stored at 2-8°C until its administration.

Suitable methods for determining the level of HC Met255 oxidation are well known to the person skilled in the art (Bertolotti-Ciarlet et al., Mol Immunol. 2009 May;46(8-9):1878-82; Liu et al., Biochemistry. 2008 May 6;47(18):5088-100). Similarly, the skilled person is also well aware of how to determine the amount of LMW species, e.g., using automated SDS-PAGE such as the 2100 Bioanalyzer System by Agilent with the Agilent Protein 230 KIT, as well as how to control it. The determination of the glycosylation pattern of an antibody is likewise standard practice, e.g., 2AB glycan mapping UPLC/HPLC.

Methods to control the deamidation level are commonly known. For example, during manufacture, the level of deamidation of a therapeutic protein may be controlled based on the day of harvest of the cells (Kaneko et al., J Biosci Bioeng. 2010 Mar;109(3):281-7), the pH and temperature during the production phase of the cell culture (Dengl et al., Pharm Res. 2013 May;30(5):1380-99) and the adjustment of the cell culture medium (Hossler et al., Biotechnol Prog. 2015 Jul-Aug;31(4):1039-52; Brühlmann et al., Biotechnol Prog. 2015 May-Jun;31(3):615-29). After manufacture, deamidation can be controlled by selecting appropriate storage conditions, including selecting an appropriate temperature and antibody formulation (Cleland et al., J Pharm Sci. 2001 Mar;90(3):310-21).

Methods for detecting and quantifying the fraction of deamidated variants are also well known to the person skilled in the art (Gervais, J Chem Technol Biotechnol 2016;91:569-575; Vlasak and lonescu, Curr Pharm Biotechnol. 2008 Dec;9(6):468-81).

Charge-related modifications, such as deamidations, are commonly detected using ion-exchange (IEX) high performance liquid chromatography (HPLC) or isoelectric focusing (IEF), but may also be detected using other chromatographic and electrophoretic techniques known to the person of skill in the art. IEX-HPLC and IEF may further be coupled with mass spectrometry (MS) techniques, such as liquid chromatography coupled MS (LC-MS) and tandem LC-MS/MS, to verify that the acidic variants identified using IEX-HPLC and IEF indeed correspond to the deamidated variants of the protein of interest.

A preferred ion-exchange chromatography is a weak cation exchange (WCX) chromatography, such as Dionex ProPac WCX-10 (also abbreviated as WCX-10). The WCX-10 column has a stationary phase composed of 10-mm non-porous, solvent compatible ethylvinylbenzenedivinylbenzene copolymer beads which are surrounded by a hydrophilic, neutral polymer. The surface of the beads is grafted with carboxylic acid groups.

According to the present invention, the proportion of deamidated variants may be determined by the following weak cation ion-exchange chromatography method:

Firstly, the sample is diluted to an avelumab antibody concentration of 5 mg/ml using Mobile Phase A (20 mM Tris-HCl, pH 5±0.1). The diluted sample is pre-treated with 5% (v/v) carboxypeptidase B. The obtained solution is transferred into an auto-sampler and may be stored refrigerated at 5 ± 3 °C for up to 72 hours. This sample is then applied to a WCX-HPLC. For instance, the following conditions of Table 1 may be applied:

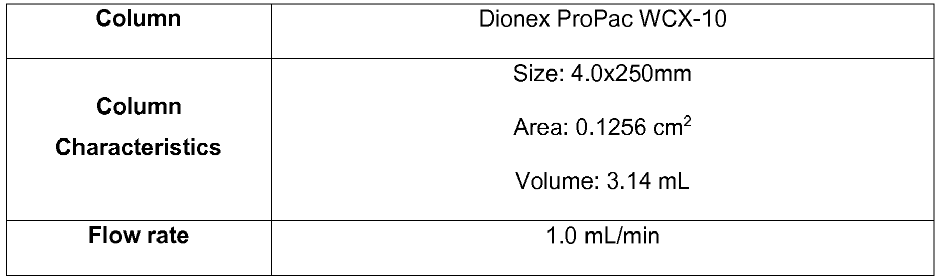

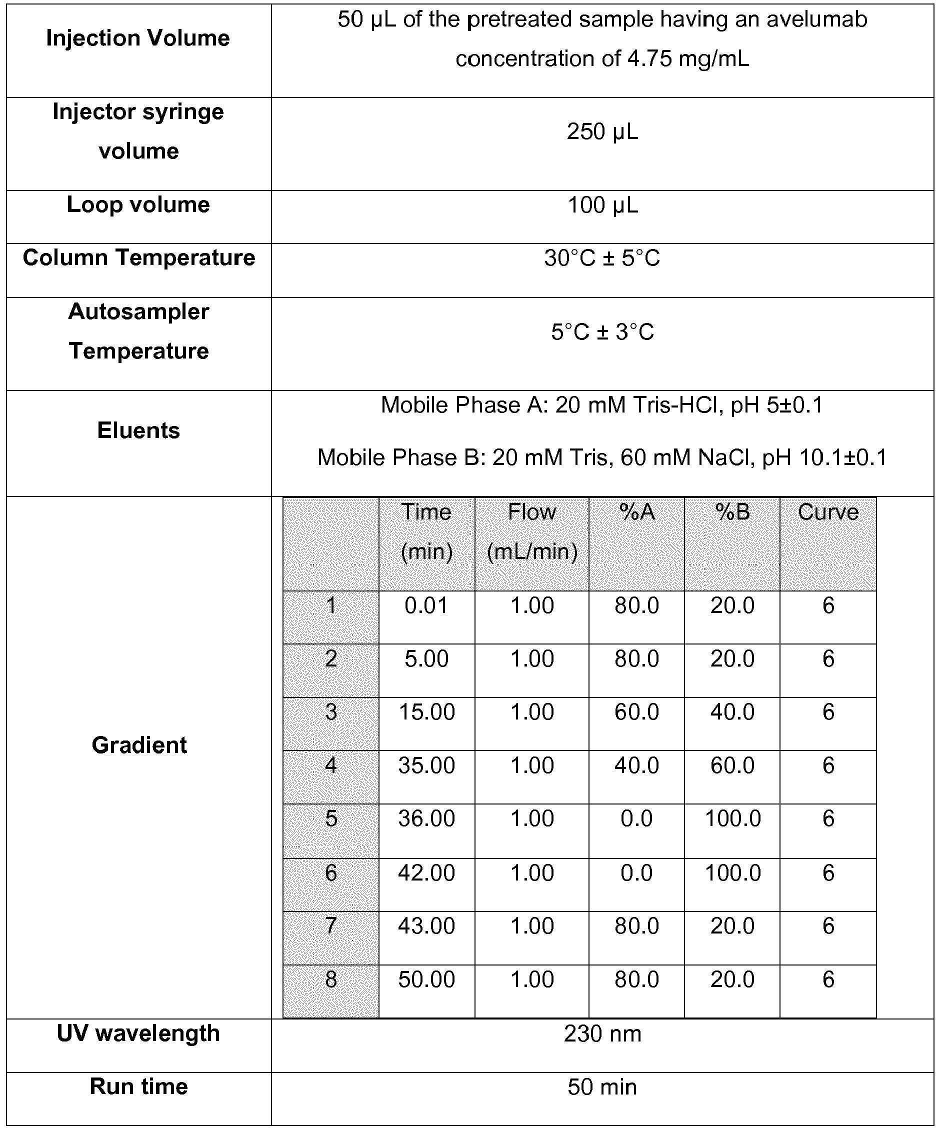

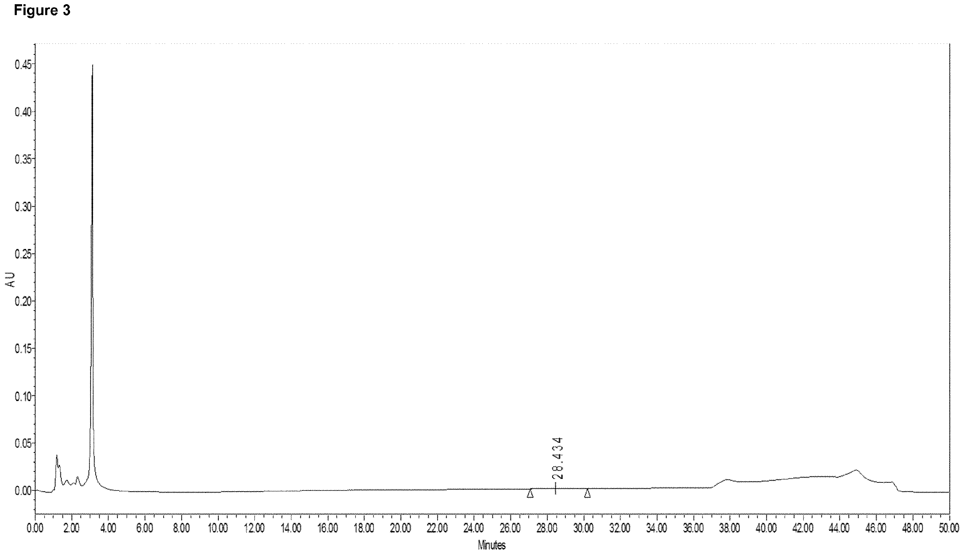

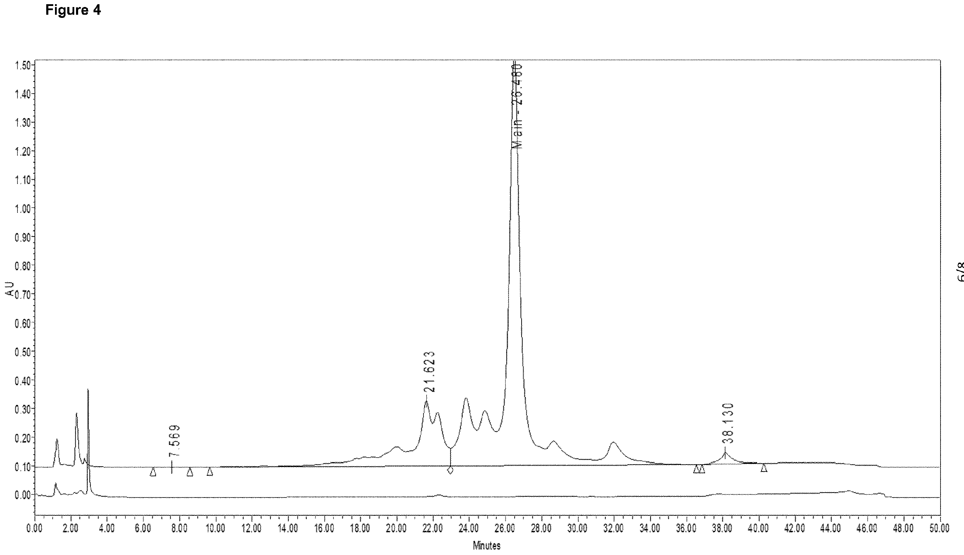

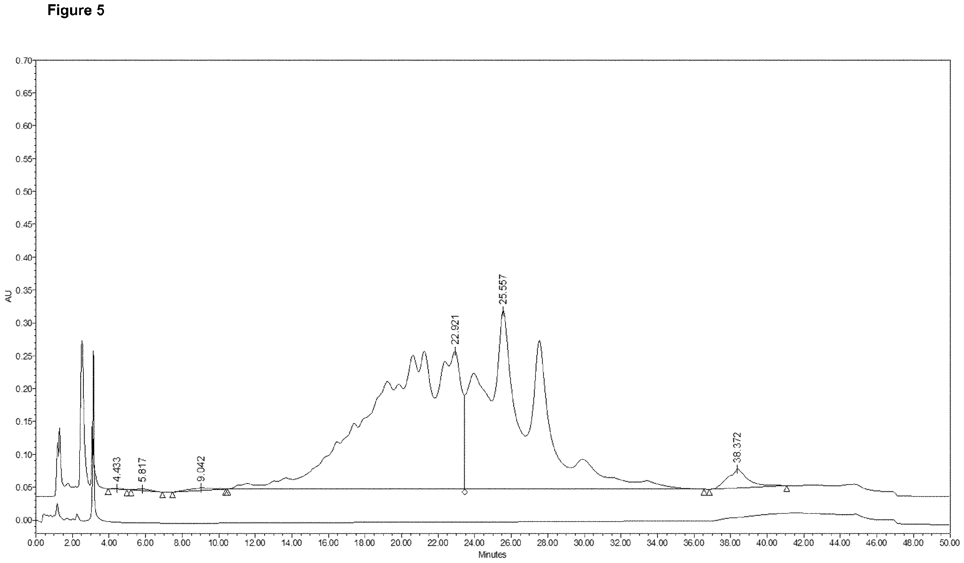

Typical chromatograms obtained with this method are shown in Figures 3 to 5.

The acidic peaks elute before the main peak. The acidic peaks in the range Rt = 6.0 min to Rt = 22.0-23.0 min predominantly relate to deamidated variants of avelumab, as confirmed by peptide mapping/MS. The proportion of the area under these peaks, as compared to the total area under the peaks corresponds to the fraction of the deamidated avelumab variants. As the skilled person readily understands, the total area under the peaks excludes the non-protein related peaks that are seen when blank sample is run (these peaks elute at about Rt = 1.0 min to about Rt = 4.0 min in the chromatogram).

In line with the foregoing, the present invention also refers to the following embodiments:
1. A composition comprising avelumab, wherein acidic species peaks elute from about retention time 6.0 minutes to about retention time 22.0-23.0 minutes in a WCX-HPLC chromatogram of the composition,
   wherein the chromatogram sample is pre-treated with carboxypeptidase B, wherein the WCX-HPLC chromatogram is generated using a first mobile phase of 20 mM Tris-HCl, pH 5±0.1 and a second mobile phase of 20 mM Tris, 60 mM NaCl, pH 10.1±0.1, and
   wherein the WCX-HPLC chromatogram is generated using detection at 230 nm.
2. The composition according to item 1, wherein the area under the curve of said acidic species peaks is more than 2%, more than 4%, more than 6%, more than 8%, more than 10% or more than 12% of the total area under the curve of the chromatogram.
3. The composition according to item 1, wherein the area under the curve of said acidic species peaks is 2-50%, 4-40%, 6-30%, 8-30%, 10-25% or 12-20% of the total area under the curve of the chromatogram.
4. The composition according to any one of items 1 to 3, wherein the chromatography is carried out under the conditions detailed in Table 1.
5. The composition according to any one of items 1 to 4, wherein the total area under the curve of the chromatogram corresponds to the peaks eluting from about retention time 6.0 minutes to about retention time 41.0-42.0 minutes.
6. The composition according to any one of items 1 to 5, wherein the stationary phase of the WCX column is composed of 10-mm non-porous, solvent compatible ethylvinylbenzenedivinylbenzene copolymer beads surrounded by a hydrophilic, neutral polymer and wherein the surface is grafted with carboxylic acid groups.
7. The composition according to item 6, wherein the the WCX column is a WCX-10 column.
8. The composition according to any one of items 1 to 7, wherein the composition exhibits any one or a combination of an increased PD-L1 binding activity on the cell surface, increased ADCC and/or increased therapeutic efficacy as compared to a composition comprising avelumab with an amount of said acidic species peaks outside said range.
9. The composition according to any one of items 1 to 8, wherein the composition has a LMW species content of less than 10%, preferably less than 8%, and more preferably less than 6%.
10. The composition according to any one of items 1 to 9, wherein the proportion of avelumab variants that are oxidated at HC Met255 is less than 10%, preferably less than 8%, and more preferably less than 6%.
11. The composition according to any one of items 1 to 10, wherein the area under the curve of said acidic species peaks is 8-25% of the total area under the curve of the chromatogram,
   wherein the composition has a LMW species content within a range of 0.1-10%; and
   wherein the proportion of avelumab variants that are oxidated at HC Met255 is within a range of 0.1-10%.
12. The composition according to any one of items 1 to 11, wherein less than 20%, preferably less than 15%, more preferably less than 10%, and most preferably less than 7.5% of the avelumab molecules are afucosylated.
13. The composition according to any one of items 1 to 12, wherein less than 20%, preferably less than 10%, more preferably less than 5%, and most preferably less than 3% of the avelumab molecules have high mannose N-glycans with five or more mannose residues.
14. A method of storage, wherein the composition according to any one of items 1 to 13 is stored after manufacture below 12°C, preferably below 10°C, more preferably below 8°C until administration to the patient.

It is also possible to quantitatively determine the amount of deamidation of specific sites and thus determine the minimal fraction of deamidated variants in the composition. The deamidation of single residues can be quantified, for instance, by petide mapping/MS. Asparagine sites that are particularly prone to deamidation include LC Asn33, LC Asn173/174, HC Asn318 and HC Asn387/392/393.

Based on peptide mapping/MS, it was found that LC Asn33 and possibly LC Asn55 of the CDR regions are prone to deamidation. The two residues may thus be responsible for the observed correlation between the overall deamidation level and cell binding activity. Accordingly, the present invention also refers to the following embodiments:
1. A composition comprising avelumab, wherein a fraction of the avelumab molecules is deamidated at Asn33 of the light chain.
2. The composition according to item 1, wherein the proportion of avelumab variants with a deamidation at Asn33 of the light chain is more than 0.5%, more than 1 %, more than 2%, more than 3%, or more than 4%.
3. The composition according to item 1 or item 2, wherein the proportion of avelumab variants with a deamidation at Asn33 of the light chain is within a range of 0.1-20%, within a range of 0.5-15%, within a range of 1-10%, within a range of 2-8%, or within a range of 3-7%.
4. A composition comprising avelumab, wherein a fraction of the avelumab molecules is deamidated at Asn55 of the light chain.
5. The composition according to item 4, wherein the proportion of avelumab variants with a deamidation at Asn55 of the light chain is more than 0.01%, more than 0.05%, or more than 0.075%.
6. The composition according to item 4 or item 5, wherein the proportion of avelumab variants with a deamidation at Asn55 of the light chain is within a range of 0.01-2%, within a range of 0.05-1%, or within a range of 0.075-0.5%.
7. The composition according to any one of items 1 to 6, wherein a fraction of the avelumab molecules is deamidated at Asn33 of the light chain and a fraction of the avelumab molecules is deamidated at Asn55 of the light chain.
8. The composition according to any one of items 1 to 7, wherein the composition exhibits any one or a combination of an increased PD-L1 binding activity on the cell surface, increased ADCC and/or increased therapeutic efficacy as compared to a composition comprising avelumab with an amount of avelumab variants with a light chain Asn33 deamidation outside said range.
9. The composition according to any one of items 1 to 8, wherein the composition has a LMW species content of less than 10%, preferably less than 8%, more preferably less than 6%.
10. The composition according to any one of items 1 to 9, wherein the proportion of avelumab variants that are oxidated at HC Met255 is less than 10%, preferably less than 8%, more preferably less than 6%.
11. The composition according to any one of items 1 to 10, wherein the proportion of avelumab variants with a deamidation at Asn33 of the light chain is within a range of 1-10%,
   wherein the composition has a LMW species content within a range of 0.1-10%; and
   wherein the proportion of avelumab variants that are oxidated at HC Met255 is within a range of 0.1-10%.
12. The composition according to any one of items 1 to 11, wherein less than 20%, preferably less than 15%, more preferably less than 10%, and most preferably less than 7.5% of the avelumab molecules are afucosylated.
13. The composition according to any one of items 1 to 12, wherein less than 20%, preferably less than 10%, more preferably less than 5%, and most preferably less than 3% of the avelumab molecules have high mannose N-glycans with five or more mannose residues.
14. An antibody comprising SEQ ID NO: 2 as the heavy chain sequence and SEQ ID NO: 1 as the light chain sequence, wherein Asn33 and/or Asn55 of SEQ ID NO: 1 is deamidated.
15. The antibody according to item 14, wherein Asn33 and/or Asn55 of SEQ ID NO: 1 is converted to aspartate or isoaspartate.
16. A method of storage, wherein the composition according to any one of items 1 to 13 or a composition comprising the antibody of item 14 or 15 is stored after manufacture below 12°C, preferably below 10°C, more preferably below 8°C until administration to the patient.

### Uses of the deamidated compositions and methods of treatment

The avelumab composition of the invention is suitable for treating a disorder in which the interaction between PD-1 and PD-L1 is detrimental.

In some embodiments, the invention provides a method of treating cancer comprising administering the avelumab composition as described above to a patient.

In an embodiment, the cancer to be treated is selected from the group consisting of non-small cell lung cancer, urothelial carcinoma, bladder cancer, mesothelioma, Merkel cell carcinoma, gastric or gastroesophageal junction cancer, ovarian cancer, breast cancer, thymoma, adenocarcinoma of the stomach, adrenocortical carcinoma, head and neck squamous cell carcinoma, renal cell carcinoma, melanoma, and/or classical Hodgkin's lymphoma.

The present invention also relates to a method of preparing an avelumab composition for transport comprising the following steps:
(a) manufacturing a plurality of compositions comprising avelumab;
(b) testing the compositions comprising avelumab to identify compositions corresponding to compositions of the invention; and
(c) introducing compositions of the invention into sterile vessels for transport.

The sterile vessels for transport can be any one selected from the group consisting of vials, syringes, ampoules, and bags.

The present invention also relates to a method for storage, wherein the composition of the invention is stored after manufacture below 12°C, preferably below 10°C, more preferably below 8°C until administration to the patient.

### Pharmaceutical formulations comprising deamidated compositions

The present invention also provides pharmaceutical formulations comprising said deamidated avelumab compositions. Such formulations preferably also comprise a pharmaceutically acceptable carrier.

Suitably, the pharmaceutical formulation is a liquid formulation or reconstituted liquid formulation. In other embodiments, the pharmaceutical formulation is present as a lyophilized product.

The concentration of the avelumab antibody in the pharmaceutical formulation may be at least 1 mg/ml, at least 5 mg/ml, at least 10 mg/ml, at least 20 mg/ml, at least 50 mg/ml or at least 100 mg/ml. In some embodiments, the concentration of the avelumab antibody is between 1 mg/ml and 100 mg/ml, between 5 mg/ml and 50 mg/ml, between 10 mg/ml and 30 mg/ml or between 15 mg/ml and 25 mg/ml. Preferably, the concentration of the avelumab antibody is about 20 mg/ml.

In one embodiment, the deamidated composition of the invention contains 20 mg/ml avelumab antibody formulated in 51 mg/ml D-mannitol, 0.6 mg/ml glacial acetic acid (100%), 0.3 mg/ml sodium hydroxide and 0.5 mg/ml polysorbate 20.

The pharmaceutical formulation may come packaged in a container, such as a vial.

### Kits comprising deamidated compositions

The present invention further provides kits comprising the deamidated avelumab compositions, preferably in a container filled with a formulation of the deamidated avelumab compositions.

Such kits suitably comprise instructions for use of the deamidated avelumab composition. Furthermore, the kits may comprise additional therapeutic reagents, such as other antibodies or small molecule anti-cancer drugs. For instance, the deamidated avelumab composition may come in a kit with cyclophosphamide, Gemcitabine, Stimuvax or core components of the FOLFOX chemotherapy.

### EXAMPLES

### Example 1: Method for quantifying deamidated variants in an avelumab composition

50 µL of avelumab sample (aqueous formulation of 20 mg/mL avelumab antibody, 51 mg/mL D-mannitol, 0.6 mg/mL glacial acetic acid, 0.3 mg/mL NaOH, 0.5 mg/mL polysorbate 20) was diluted with 150 µL Mobile Phase A (20 mM Tris-HCl, pH 5±0.1). 10 µL of Carboxypeptidase B (npCarboxyxpeptidase B, order code 2500, ASA Spezialenzyme GmbH) was added and transferred to a refrigerated auto sampler (5°C ± 3°C).

50 µL of the pre-treated sample was injected into the IEX-HPLC under the conditions set out in Table 1 above.

The fraction of deamidated avelumab variants was calculated based on the area under the peaks of the peaks corresponding to the deamidated variants (Rt = 6.0 min to Rt = 22.0-23.0 min), as compared to the total area under the peaks.

### Example 2: Method for quantifying the cell binding activity of avelumab

The biological activity of avelumab was evaluated through a cell based assay able to measure its capability to bind the human PD-L1 receptor over-expressed on a recombinant HEK-293 (hPD-L1) cell line. To this end, avelumab antibodies of the avelumab composition to be tested were allowed to bind to a protein A-coated 96-well plate for 30 minutes at room temperature under swirling. Then, 50,000 HEK-293 (hPD-L1) cells were added per well of the anti-PD-L1 antibody coated plate and allowed to bind to the anti-PD-L1 antibodies for 1 hour at 37°C, 5% CO2. The unbound cells were washed away and presence of the bound cells was confirmed in each well by the ATPlite 1 step (Perkin Elmer). Counts per seconds are then plotted against the Log transformed anti-PDL-1 antibody concentration and fitted by 4PL (sigmoidal dose response curve). For each data set, the concentration of anti PDL-1 antibody able to bind at 50% of the maximum possible PDL-1 binding capacity (EC50) is calculated. The biological activity of a sample is expressed as % activity with respect to reference material and is the percentage expression of the potency ratio (i.e., the EC50 ratio of equipotent doses of standard and unknown preparations). The Potency is released as the average results coming from three independent assays.

### Example 3: Biological activity of artificially deamidated avelumab samples

The biological activity of avelumab samples with different deamidation levels was tested. Samples were artificially deamidated by treating them under stressed (incubation at 50°C) or accelerated conditions (incubation at 25°C).

The proportion of deamidated avelumab variants was then determined in accordance with the method outlined in Example 1 and the level of biological activity was measured in accordance with the method outlined in Example 2.

The results were as follows:

| **Batch no.** | **Time (months)** | **Treatment conditions** | **Level of deamidation (%)** | **Cell binding activity (%)** |
|---|---|---|---|---|
| 1 | 0 | Stressed | 13.9 | 92 |
| 1 | 1 | Stressed | 43.9 | 119 |
| 2 | 0 | Stressed | 13.2 | 91 |
| 2 | 1 | Stressed | 44.3 | 115 |
| 3 | 0 | Stressed | 13.1 | 88 |
| 3 | 1 | Stressed | 40.9 | 109 |
| 4 | 0 | Stressed | 14.39 | 101 |
| 4 | 1 | Stressed | 21.21 | 102 |
| 4 | 2 | Stressed | 37.6 | 91 |
| 4 | 3 | Stressed | 37.88 | 96 |
| 5 | 0 | Stressed | 13.58 | 105 |
| 5 | 1 | Stressed | 21.49 | 99 |
| 5 | 2 | Stressed | 37.99 | 104 |
| 5 | 3 | Stressed | 39.18 | 109 |
| 4 | 0 | Accelerated | 14.4 | 101 |
| 4 | 1 | Accelerated | 14.4 | 104 |
| 4 | 2 | Accelerated | 16.5 | 82 |
| 4 | 3 | Accelerated | 17.2 | 97 |
| 4 | 6 | Accelerated | 19.9 | 90 |
| 5 | 0 | Accelerated | 13.6 | 105 |
| 5 | 1 | Accelerated | 14.5 | 95 |
| 5 | 2 | Accelerated | 16.5 | 102 |
| 5 | 3 | Accelerated | 17.4 | 96 |
| 5 | 6 | Accelerated | 19.9 | 93 |
| 6 | 0 | Accelerated | 14.2 | 94 |
| 6 | 1 | Accelerated | 14.4 | 98 |
| 6 | 2 | Accelerated | 16.4 | 88 |
| 6 | 3 | Accelerated | 17.1 | 94 |
| 6 | 6 | Accelerated | 19.9 | 99 |

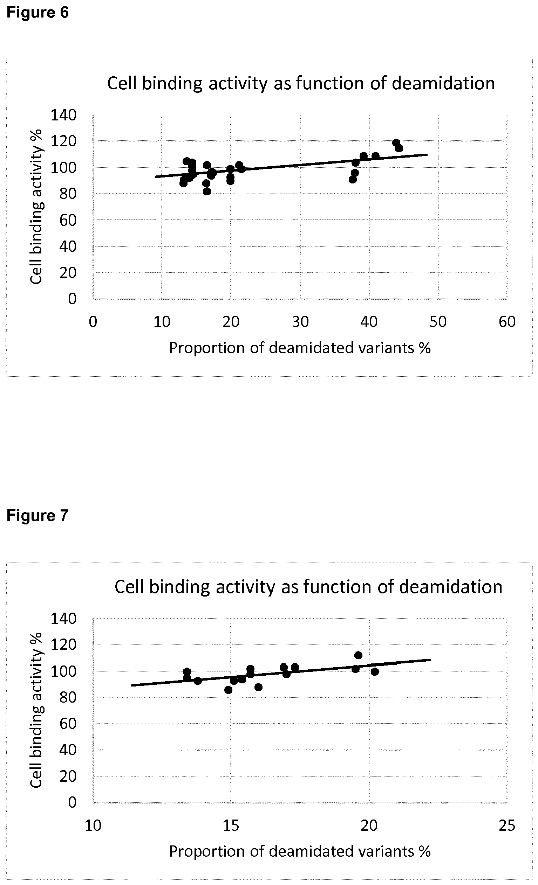

The proportion of deamidated variants was plotted against the cell binding activity and there was a significant correlation found between them with a P value of 0.0014 (Figure 6).

### Example 4: Biological activity of artificially deamidated avelumab samples

The biological activity of avelumab samples with different deamidation levels was also tested in another set of experiments. Samples were artificially deamidated by treating them under accelerated conditions (incubation at 25°C).

The proportion of deamidated avelumab variants was then determined in accordance with the method outlined in Example 1 and the level of biological activity was measured in accordance with the method outlined in Example 2.

The results were as follows:

| **Batch no.** | **Time (months)** | **Treatment conditions** | **Level of deamidation (%)** | **Cell binding activity (%)** |
|---|---|---|---|---|
| 7 | 0 | Accelerated | 13.8 | 93 |
| 7 | 1 | Accelerated | 15.4 | 94 |
| 7 | 2 | Accelerated | 16 | 88 |
| 7 | 3 | Accelerated | 17.3 | 103 |
| 7 | 6 | Accelerated | 20.2 | 100 |
| 8 | 0 | Accelerated | 13.4 | 100 |
| 8 | 1 | Accelerated | 15.1 | 93 |
| 8 | 2 | Accelerated | 15.7 | 98 |
| 8 | 3 | Accelerated | 16.9 | 103 |
| 8 | 6 | Accelerated | 19.6 | 112 |
| 9 | 0 | Accelerated | 13.4 | 95 |
| 9 | 1 | Accelerated | 14.9 | 86 |
| 9 | 2 | Accelerated | 15.7 | 102 |
| 9 | 3 | Accelerated | 17 | 98 |
| 9 | 6 | Accelerated | 19.5 | 102 |

The proportion of deamidated variants was plotted against the cell binding activity and there was a significant correlation found between them with a P value of 0.0220 (Figure 7).

## Claims

1. A composition comprising avelumab, wherein a fraction of the avelumab molecules is deamidated.

2. The composition according to claim 1, wherein the proportion of deamidated avelumab variants is more than 6%, preferably more than 8%.

3. The composition according to claim 2, wherein the proportion of deamidated avelumab variants is within a range of 6-30%, preferably within a range of 8-25%.

4. The composition according to any one of claims 1 to 3, wherein the composition exhibits any one or a combination of an increased PD-L1 binding activity on the cell surface, increased ADCC and/or increased therapeutic efficacy as compared to a composition comprising avelumab with an amount of deamidated variants outside said range.

5. The composition according to any one of claims 1 to 4, wherein the composition has a LMW species content of less than 10%, preferably less than 8%, more preferably less than 6%.

6. The composition according to any one of claims 1 to 5, wherein the proportion of avelumab variants that are oxidated at HC Met255 is less than 10%, preferably less than 8%, more preferably less than 6%.

7. The composition according to any one of claims 1 to 6, wherein the proportion of deamidated avelumab variants is within a range of 8-25%,
wherein the composition has a LMW species content within a range of 0.1-10%; and
wherein the proportion of avelumab variants that are oxidated at HC Met255 is within a range of 0.1-10%.

8. The composition according to any one of claims 1 to 7, wherein less than 20%, preferably less than 15%, more preferably less than 10%, and most preferably less than 7.5% of the avelumab molecules are afucosylated.

9. The composition according to any one of claims 1 to 8, wherein less than 20%, preferably less than 10%, more preferably less than 5%, and most preferably less than 3% of the avelumab molecules have high mannose N-glycans with five or more mannose residues.

10. The composition according to any one of claims 1 to 9 for use as a medicament.

11. The composition according to any one of claims 1 to 9 for use in the treatment of a disorder in which the interaction between PD-1 and PD-L1 is detrimental.

12. The composition according to any one of claims 1 to 9 for use in the treatment of cancer.

13. A method of treating cancer comprising administering a composition according to any one of claims 1 to 9.

14. The composition for use according to claim 12 or the method of claim 13, wherein the cancer is selected from the group consisting of non-small cell lung cancer, urothelial carcinoma, bladder cancer, mesothelioma, Merkel cell carcinoma, gastric or gastroesophageal junction cancer, ovarian cancer, breast cancer, thymoma, adenocarcinoma of the stomach, adrenocortical carcinoma, head and neck squamous cell carcinoma, renal cell carcinoma, melanoma, and/or classical Hodgkin's lymphoma.

15. A pharmaceutical composition comprising the composition according to any one of claims 1 to 9 and a pharmaceutically-acceptable carrier.

16. A kit comprising the pharmaceutical composition according to claim 15 and instructions for use.

17. A method of storage, wherein the composition according to any one of claims 1 to 9, the pharmaceutical composition according to claim 15 or the kit according to claim 16 is stored after manufacture below 12°C, preferably below 10°C, more preferably below 8°C until administration to the patient.

18. A method of preparing an avelumab composition for transport, the method comprising:
(a) manufacturing a plurality of compositions comprising avelumab;
(b) testing the compositions comprising avelumab to identify compositions wherein more than 6%, preferably more than 8% of the avelumab is deaminated; and
(c) introducing compositions identified as having more than 6% preferably more than 8% of the avelumab deaminated into sterile vessels for transport.

19. A method of preparing an avelumab composition for transport, the method comprising:
(a) manufacturing a plurality of compositions comprising avelumab;
(b) testing the compositions comprising avelumab to identify compositions wherein 6-30%, preferably 8-25% of the avelumab is deaminated; and
(c) introducing compositions identified as having 6-30%, preferably 8-25% of the avelumab deaminated into sterile vessels for transport.

20. A method according to claim 18 or 19, wherein the sterile vessels are selected from the group consisting of vials, syringes, ampoules, and bags.
